# EUROPEAN PATENT APPLICATION

(11) **EP 4 772 619 A1**
(43) Date of publication of application: **08.07.2026**
(21) Application number: 23955863.8
(22) Date of filing: 31.10.2023
(51) Int. Cl.: C12N 9/10, C12N 15/54, C12P 19/02

(54) **PYRIMIDINE NUCLEOSIDE PHOSPHORYLASE MUTANT AND PREPARATION METHOD FOR 2'-FLUORO NUCLEOSIDE**

(30) Priority: 17.10.2023 CN 202311341031
(71) Applicant: Jilin Asymchem Pharmaceuticals Co., Ltd., Dunhua, Yanbian, Jilin 133710 (CN); Tianjin Asymchem Biotechnology Co., Ltd., Tianjin 300457 (CN)
(72) Inventor: HONG, Hao, Morrisville North Carolina 27560 (US); JAMES, Gage, Morrisville North Carolina 27560 (US); ZHANG, Na, Tianjin 300457 (CN); JIAO, Xuecheng, Tianjin 300457 (CN); LI, Mingji, Tianjin 300457 (CN); LI, Rui, Tianjin 300457 (CN); CUI, Yi, Tianjin 300457 (CN); CHENG, Yibing, Tianjin 300457 (CN); ZHANG, Haibin, Tianjin 300457 (CN); DING, Shimao, Tianjin 300457 (CN); MI, Jiali, Tianjin 300457 (CN); GAO, Qiuyue, Tianjin 300457 (CN); DAI, Hongqian, Tianjin 300457 (CN)
(74) Representative: Dehns Germany Partnerschaft mbB
(86) International application number: PCT/CN2023/128846
(87) International publication number: WO 2025/081545

(57) **Abstract**

Provide is a pyrimidine nucleoside phosphorylase mutant and a method for preparing a 2'-fluoronucleoside. The pyrimidine nucleoside phosphorylase mutant includes: (a) a protein with a mutation based on the pyrimidine nucleoside phosphorylase wild-type enzyme TtPyNP as shown in SEQ **ID** NO: 1, where the mutation is selected from any one or more of the following mutations: K80, etc.; or (b) a protein that has greater than 70% identity to the amino acid sequence as defined in (a) and has a pyrimidine nucleoside phosphorylase activity. The present application solves the problem of low yield in enzyme-catalyzed synthesis of 2'-fluoronucleoside in the prior art and is suitable for the field of enzyme catalysis.

## Description

This application is based upon and claims priority to Chinese Patent Application No. 202311341031.9, filed on October 17, 2023, the disclosure of which is hereby incorporated by reference in its entirety.

### Technical Field

The present application relates to the field of enzyme catalysis, and specifically to a pyrimidine nucleoside phosphorylase mutant and a method for preparing 2'-fluoronucleoside.

### Background

Oligonucleotide drugs include antisense oligonucleotides (ASO), small interfering RNA (siRNA), aptamers, etc. Their active ingredients are mainly RNA and DNA, which can specifically act on targets according to the principle of complementary base pairing. Currently, more than 10 types of oligonucleotide drugs have been approved for marketing, and are applied in the field of ophthalmology, as well as in the field of genetic diseases such as cardiovascular and metabolic diseases. Clinical experiments have shown that oligonucleotide drugs can also be applied in many fields such as cancer, neurological diseases, respiratory diseases, and infectious diseases, thus having broad application prospects. Oligonucleotide drugs often require specific chemical modifications to improve their stability and extend their half-life in vivo, such as fluorination modification at the 2'-position of ribose. Therefore, with the continuous expansion of the market scale of oligonucleotide drugs, the market demand for 2'-fluorinated nucleoside monomers, which are important synthetic precursors of such drugs, is also increasing steadily.

Currently, 2'-fluoronucleoside monomers on the market are produced by chemical methods. However, the chemical synthesis process is relatively cumbersome, involving multiple steps, requiring protection and deprotection of multiple functional groups, and the use of organic reagents that cause environmental pollution, resulting in high production costs. In addition, 2'-fluorinated nucleoside monomers can also be synthesized by biological methods. For example, pyrimidine nucleoside phosphorylase (PyNP) and purine nucleoside phosphorylase (PNP) can be utilized to synthesize 2'-fluoro-2'-deoxyadenosine from 2'-fluoro-2'-deoxyuridine and adenine through a one-pot method. However, due to the poor enzyme activity of the reaction catalyzed by pyrimidine nucleoside phosphorylase (PyNP) for converting 2'-fluoro-2'-deoxyuridine into 2'-fluoro-2'-deoxyribose-1-phosphate and uracil, the commercial production of 2'-fluorinated nucleoside monomers with biological methods is limited. Szeker et al. utilized the wild-type pyrimidine nucleoside phosphorylase derived from *Geobacillus thermoglucosidasius* (GsPyNP), and only 0.004 mM of 2'-fluoro-2'-deoxyuridine could be catalyzed within 0.5 h, while the wild-type pyrimidine nucleoside phosphorylase derived from *Thermus thermophilus* (TtPyNP) could hydrolyze 0.156 mM of 2'-fluoro-2'-deoxyuridine. Under optimal conditions, only 0.65 mM of 2'-fluoro-2'-deoxyuridine could be catalyzed with the wild-type TtPyNP after 17 h of reaction (Comparative investigations on thermostable pyrimidine nucleoside phosphorylases from *Geobacillus thermoglucosidasius* and *Thermus thermophilus).*

### Summary

A main objective of the present application is to provide a pyrimidine nucleoside phosphorylase mutant and a method for preparing 2'-fluoronucleoside, so as to solve the problem of low yield in enzyme-catalyzed synthesis of 2'-fluoronucleoside in the prior art.

To achieve the above objective, according to a first aspect of the present application, a pyrimidine nucleoside phosphorylase mutant is provided, including: (a) a protein with a mutation based on the pyrimidine nucleoside phosphorylase wild-type enzyme TtPyNP as shown in SEQ ID NO: 1, where the mutation is selected from any one or more of the following mutations: K80, V4, I7, R8, R17, E19, G27, R30, P34, L47, G49, L50, L58, D61, S91, F105, G112, T119, E135, A151, A157, V173, I179, I183, A189, A190, F206, M207, L215, G224, Q225, A227, V231, E251, A271, L274, R280, L281, L284, G285, R303, D329, V337, G355 or K360; or (b) a protein that has greater than 70% identity to the amino acid sequence as defined in (a) and has a pyrimidine nucleoside phosphorylase activity.

Further, in the (a), a type of amino acid substituted at each site is each independently selected from the following:
K80Q, K80Y, V4P, V4G, I7G, R8N, R8F, R17E, E19P, G27S, R30K, P34K, P34L, P34G, L47H, G49T, G49V, G49S, L50A, L50D, L50G, L50K, L50N, L50R, L50S, L58T, L58Q, D61T, S91A, S91V, S91C, F1051, F105T, G112S, G112T, G112A, G112L, G112C, T119N, E135K, E135A, A151S, A157D, A157N, A157T, V173S, V173T, I179V, I183V, A189V, A189T, A190T, A190V, A1901, F206M, M207L, M207R, L2151, G224D, Q225N, A227D, V231D, E251V, A271S, L274K, L2741, R280V, L281R, L284S, L284G, G285D, R303K, R303A, D329F, V337T, G355S, K360L or K360I;
where a letter before a number represents an original amino acid, and a letter after the number represents a mutated amino acid.

Further, the mutation includes any one of the following amino acid mutations:
K80Q+G112A, K80Q+G112C, K80Q+G112S, K80Q+G112T, L50A+K80Q+G112T, L50D+K80Q+G112T, L50G+K80Q+G112T, L50K+K80Q+G112T, L50N+K80Q+G112T, L50S+K80Q+G112T, K80Q+G112T+A157T, D61T+K80Q+G112T+A157T, E19P+K80Q+G112T+A157T, G27S+K80Q+G112T+A157T+L281R, G49S+K80Q+G112T+A157T, G49T+K80Q+G112T+A157T, G49V+K80Q+G112T+A157T, I7G+K80Q+G112T+A157T, K80Q+G112T+A157T+A190T+L281R, K80Q+G112T+A157T+A190V+L281R, K80Q+G112T+A157T+A271S+L281R, K80Q+G112T+A157T+L215I+L281R, K80Q+G112T+A157T+L274I+L281R, K80Q+G112T+A157T+L274K, K80Q+G112T+A157T+L281R, K80Q+G112T+A157T+L281R+G355S, K80Q+G112T+A157T+L281R+K360I, K80Q+G112T+A157T+L281R+V337T, K80Q+G112T+A157T+L284G, K80Q+G112T+A157T+L284S, K80Q+G112T+A157T+Q225N+L281R, K80Q+G112T+A157T+R280V, K80Q+G112T+A157T+R303K, K80Q+G112T+A157T+V173S, K80Q+G112T+A157T+V173T+A189V+L281R, K80Q+G112T+A157T+V173T+L281R, K80Q+G112T+A189V, K80Q+G112T+A190I, K80Q+G112T+A190T, K80Q+G112T+E135A+A157T+L281R, K80Q+G112T+E135K, K80Q+G112T+K360I, K80Q+G112T+V173T, L47H+K80Q+G112T+A157T, L50A+K80Q+G112T+A157T+V173T+L281R, L50K+K80Q+G112T+A157T+L281R, L50N+K80Q+G112T+A157T+L281R, L50R+K80Q+G112T+A157T, L58Q+K80Q+G112T+A157T, L58T+K80Q+G112T+A157T, P34G+K80Q+G112T+A157T, P34K+K80Q+G112T+A157T, P34L+K80Q+G112T+A157T+L281R, R17E+K80Q+G112T+A157T, VAG+K80Q+G112T+A157T+L281R, V4P+K80Q+G112T+A157T, V4P+L50R+G112C+I183V+A190T+L2151+L281R+K360L, E19P+P34K+F105I+A157N+V173S+F206M+V231D+L281R, K80Q+G112T+A157T+V173T+A227D+L281R, K80Q+G112T+A157T+V173T+G224D+L281R, K80Q+G112T+A157T+V173T+L281R+D329F, K80Q+G112T+A157T+V173T+L281R+G285D, K80Q+G112T+A157T+V173T+L281R+R303A, L47H+L58Q+S91V+T119N+A157T+M207R+G224D+A271S, R17E+R30K+G112L+E135K+A157D+I183V+L284G+K360I, R30K+K80Q+G112T+A157T+V173T+L281R, R8F+G27S+S91A+G112T+A151S+V173T+M207L+V337T, V4G+R8N+P34L+G49V+L50D+V173T+L2151+G355S, K80Q+G112S+I179V+A227D+L274K+R280V+R303A+K360L, K80Q+G112T+A157T+V173T+A189T+A227D+L281R, K80Q+G112T+A157T+V173T+A189V+A227D+L281R, K80Q+G112T+A157T+V173T+A190V+A227D+L281R, K80Q+G112T+A157T+V173T+F206M+A227D+L281R, K80Q+G112T+A157T+V173T+1179V+A227D+L281R, K80Q+G112T+A157T+V173T+I183V+A227D+L281R, K80Q+G112T+A157T+V173T+M207R+A227D+L281R, K80Q+G112T+T119N+A157T+V173T+A227D+L281R, K80Y+F105T+G112A+A157T+V173T+A189T+Q225N+L2741, L50A+K80Q+V173T+I183V+A189T+Q225N+L274I+G285D, L50G+K80Y+T119N+V173T+L215I+L284S+R303K+D329F, K80Q+G112T+T119N+A157T+V173T+I179V+A227D+E251V, K80Q+G112T+T119N+A157T+V173T+I179V+A227D+L281R, K80Q+G112T+T119N+A157T+V173T+I183V+A227D+L281R, K80Q+G112T+T119N+A157T+V173T+A189T+A227D+L281R, K80Q+G112T+T119N+A157T+V173T+A189V+A227D+L281R, K80Q+G112T+T119N+A157T+V173T+A190T+A227D+L281R, K80Q+G112T+T119N+A157T+V173T+A190V+A227D+L281R , K80Q+G112T+T119N+A157T+V173T+F206M+A227D+L281R, or K80Q+G112T+T119N+A157T+V173T+M207L+A227D+L281R.

Further, the pyrimidine nucleoside phosphorylase mutant includes a protein that has greater than 75%, greater than 80%, greater than 85%, more preferably greater than 95%, and further preferably greater than 99% identity to the amino acid sequence as defined in (a) and has a pyrimidine nucleoside phosphorylase activity.

To achieve the above objective, according to a second aspect of the present application, a DNA molecule is provided, where the DNA molecule encodes the above pyrimidine nucleoside phosphorylase mutant.

To achieve the above objective, according to a third aspect of the present application, a recombinant plasmid is provided, where the above DNA molecule is ligated into the recombinant plasmid.

To achieve the above objective, according to a fourth aspect of the present application, a host cell is provided, where the host cell includes the DNA molecule or the recombinant plasmid.

Further, the host cell includes a prokaryotic cell; and preferably, the prokaryotic cell includes *Escherichia coli.*

To achieve the above objective, according to a fifth aspect of the present application, a method for preparing a 2'-fluoronucleoside is provided, where the method includes: a) catalyzing the cleavage of a substrate nucleoside into a phosphorylated 2'-fluoropentose and a free base with the above pyrimidine nucleoside phosphorylase mutant, where the substrate nucleoside has a 2'-fluoropentose structure; b) catalyzing the binding of the phosphorylated 2'-fluoropentose to a substrate base with a purine nucleoside phosphorylase, so as to obtain the 2'-fluoronucleoside.

Further, the substrate nucleoside includes a 2'-fluoro-2'-deoxyuridine, a 2'-fluoro-2'-deoxycytidine or a 2'-deoxy-2'-fluorothymidine.

Further, the substrate base includes a modified or unmodified adenine, guanine, thymine or cytosine, or a modified uracil.

Further, the 2'-fluoronucleoside includes a nucleoside containing a 2'-fluoro-2'-deoxypentose structure, and preferably, the 2'-fluoronucleoside includes a 2'-fluoro-2'-deoxyadenosine, a 2'-fluoro-2'-deoxy-2-aminoadenosine, a 2'-fluoro-2'-deoxyguanosine or a 2'-fluoro-2'-deoxycytidine; or the 2'-fluoronucleoside is selected from 2'-fluoro-2'-deoxynucleosides with substituents on the base structure.

To achieve the above objective, according to a sixth aspect of the present application, a method for preparing a phosphorylated 2'-fluoropentose is provided, where the method includes: catalyzing the cleavage of a substrate nucleoside into a phosphorylated 2'-fluoropentose and a free base with the above pyrimidine nucleoside phosphorylase mutant, where the substrate nucleoside has a 2'-fluoropentose structure.

Further, the substrate nucleoside includes a 2'-fluoro-2'-deoxyuridine, a 2'-fluoro-2'-deoxycytidine or a 2'-deoxy-2'-fluorothymidine.

By applying the technical solutions of the present application, the substrate nucleoside containing a 2'-fluoropentose structure can be catalyzed to cleave into a phosphorylated 2'-fluoropentose and a free base with the above pyrimidine nucleoside phosphorylase mutant, thereby preparing and obtaining the target product 2'-fluoronucleoside. Compared with the wild-type enzyme, the above pyrimidine nucleoside phosphorylase mutant has a better enzyme activity, and a higher conversion rate and greater efficiency in preparing 2'-fluoronucleoside products. It can significantly reduce the amount of enzyme used, thereby lowering production costs, and is suitable for industrial scale-up production.

### Brief Description of the Drawings

The drawings that form part of this application are used to provide a further understanding of the present application. The schematic examples of the present application and their descriptions are used to explain the present application, and do not constitute an improper limitation of the present application. In the drawings:
Fig. 1 shows a schematic diagram of a chemical reaction in which a substrate nucleoside with a 2'-fluoropentose is catalyzed to cleave into a phosphorylated 2'-fluoropentose and a free base with a pyrimidine nucleoside phosphorylase according to an embodiment of the present application.
Fig. 2 shows a schematic diagram of the chemical reaction in which binding of a phosphorylated 2'-fluoropentose and a substrate base is catalyzed with a purine nucleoside phosphorylase to prepare and obtain 2'-fluoronucleoside according to an embodiment of the present application.
Fig. 3 shows an HPLC chromatogram of preparing 2'-fluoro-2'-deoxyadenosine with 2'-fluoro-2'-deoxyuridine and adenine as substrates according to Example 6 of the present application.

### Detailed Description of the Embodiments

It should be noted that, in the absence of a conflict, embodiments of this application can be combined with features in the embodiments. The present application will be described in detail below in conjunction with the embodiments.

Terminology explanation:
2'-fluoronucleoside: a nucleoside in which the hydroxyl group on the 2-position carbon atom of the pentose is replaced by a fluorine atom.

As mentioned in the background, in the prior art, the yield and productivity of preparing 2'-fluoronucleoside with enzyme catalysis are relatively low, which is difficult to meet the requirements of industrial production. Therefore, in this application, the inventors attempt to develop a pyrimidine nucleoside phosphorylase mutant with a higher enzyme activity, which can efficiently prepare the target product 2'-fluoronucleoside, thus proposing a series of protection schemes in this application.

In a first typical implementation of this application, a pyrimidine nucleoside phosphorylase mutant is provided, including: (a) a protein with a mutation based on the pyrimidine nucleoside phosphorylase wild-type enzyme TtPyNP as shown in SEQ ID NO: 1, where the mutation is selected from any one or more of the following mutations: K80, V4, 17, R8, R17, E19, G27, R30, P34, L47, G49, L50, L58, D61, S91, F105, G112, T119, E135, A151, A157, V173, I179, 1183, A189, A190, F206, M207, L215, G224, Q225, A227, V231, E251, A271, L274, R280, L281, L284, G285, R303, D329, V337, G355 or K360; or
(b) a protein that has greater than 70% identity to the amino acid sequence as defined in (a) and has a pyrimidine nucleoside phosphorylase activity.

In a preferred embodiment, in the above (a), a type of amino acid substituted at each site is each independently selected from the following:
K80Q, K80Y, V4P, V4G, I7G, R8N, R8F, R17E, E19P, G27S, R30K, P34K, P34L, P34G, L47H, G49T, G49V, G49S, L50A, L50D, L50G, L50K, L50N, L50R, L50S, L58T, L58Q, D61T, S91A, S91V, S91C, F1051, F105T, G112S, G112T, G112A, G112L, G112C, T119N, E135K, E135A, A151S, A157D, A157N, A157T, V173S, V173T, I179V, I183V, A189V, A189T, A190T, A190V, A1901, F206M, M207L, M207R, L2151, G224D, Q225N, A227D, V231D, E251V, A271S, L274K, L2741, R280V, L281R, L284S, L284G, G285D, R303K, R303A, D329F, V337T, G355S, K360L or K360I; where a letter in front of a number represents an original amino acid, and a letter behind the number represents a mutated amino acid.

In a preferred embodiment, the mutation includes any one of the following amino acid mutations: K80Q+G112A, K80Q+G112C, K80Q+G112S, K80Q+G112T, L50A+K80Q+G112T, L50D+K80Q+G112T, L50G+K80Q+G112T, L50K+K80Q+G112T, L50N+K80Q+G112T, L50S+K80Q+G112T, K80Q+G112T+A157T, D61T+K80Q+G112T+A157T, E19P+K80Q+G112T+A157T, G27S+K80Q+G112T+A157T+L281R, G49S+K80Q+G112T+A157T, G49T+K80Q+G112T+A157T, G49V+K80Q+G112T+A157T, I7G+K80Q+G112T+A157T, K80Q+G112T+A157T+A190T+L281R, K80Q+G112T+A157T+A190V+L281R, K80Q+G112T+A157T+A271S+L281R, K80Q+G112T+A157T+L215I+L281R, K80Q+G112T+A157T+L274I+L281R, K80Q+G112T+A157T+L274K, K80Q+G112T+A157T+L281R, K80Q+G112T+A157T+L281R+G355S, K80Q+G112T+A157T+L281R+K360I, K80Q+G112T+A157T+L281R+V337T, K80Q+G112T+A157T+L284G, K80Q+G112T+A157T+L284S, K80Q+G112T+A157T+Q225N+L281R, K80Q+G112T+A157T+R280V, K80Q+G112T+A157T+R303K, K80Q+G112T+A157T+V173S, K80Q+G112T+A157T+V173T+A189V+L281R, K80Q+G112T+A157T+V173T+L281R, K80Q+G112T+A189V, K80Q+G112T+A190I, K80Q+G112T+A190T, K80Q+G112T+E135A+A157T+L281R, K80Q+G112T+E135K, K80Q+G112T+K360I, K80Q+G112T+V173T, L47H+K80Q+G112T+A157T, L50A+K80Q+G112T+A157T+V173T+L281R, L50K+K80Q+G112T+A157T+L281R, L50N+K80Q+G112T+A157T+L281R, L50R+K80Q+G112T+A157T, L58Q+K80Q+G112T+A157T, L58T+K80Q+G112T+A157T, P34G+K80Q+G112T+A157T, P34K+K80Q+G112T+A157T, P34L+K80Q+G112T+A157T+L281R, R17E+K80Q+G112T+A157T, VAG+K80Q+G112T+A157T+L281R, V4P+K80Q+G112T+A157T, V4P+L50R+G112C+1183V+A190T+L2151+L281R+K360L, E19P+P34K+F105I+A157N+V173S+F206M+V231D+L281R, K80Q+G112T+A157T+V173T+A227D+L281R, K80Q+G112T+A157T+V173T+G224D+L281R, K80Q+G112T+A157T+V173T+L281R+D329F, K80Q+G112T+A157T+V173T+L281R+G285D, K80Q+G112T+A157T+V173T+L281R+R303A, L47H+L58Q+S91V+T119N+A157T+M207R+G224D+A271S, R17E+R30K+G112L+E135K+A157D+1183V+L284G+K360I, R30K+K80Q+G112T+A157T+V173T+L281R, R8F+G27S+S91A+G112T+A151S+V173T+M207L+V337T, V4G+R8N+P34L+G49V+L50D+V173T+L2151+G355S, K80Q+G112S+I179V+A227D+L274K+R280V+R303A+K360L, K80Q+G112T+A157T+V173T+A189T+A227D+L281R, K80Q+G112T+A157T+V173T+A189V+A227D+L281R, K80Q+G112T+A157T+V173T+A190V+A227D+L281R, K80Q+G112T+A157T+V173T+F206M+A227D+L281R, K80Q+G112T+A157T+V173T+I179V+A227D+L281R, K80Q+G112T+A157T+V173T+I183V+A227D+L281R, K80Q+G112T+A157T+V173T+M207R+A227D+L281R, K80Q+G112T+T119N+A157T+V173T+A227D+L281R, K80Y+F105T+G112A+A157T+V173T+A189T+Q225N+L2741, L50A+K80Q+V173T+I183V+A189T+Q225N+L274I+G285D, L50G+K80Y+T119N+V173T+L215I+L284S+R303K+D329F, K80Q+G112T+T119N+A157T+V173T+I179V+A227D+E251V, K80Q+G112T+T119N+A157T+V173T+I179V+A227D+L281R, K80Q+G112T+T119N+A157T+V173T+I183V+A227D+L281R, K80Q+G112T+T119N+A157T+V173T+A189T+A227D+L281R, K80Q+G112T+T119N+A157T+V173T+A189V+A227D+L281R, K80Q+G112T+T119N+A157T+V173T+A190T+A227D+L281R, K80Q+G112T+T119N+A157T+V173T+A190V+A227D+L281R , K80Q+G112T+T119N+A157T+V173T+F206M+A227D+L281R, or K80Q+G112T+T119N+A157T+V173T+M207L+A227D+L281R.

Or a protein that has greater than 70%, 75%, 80%, 85%, 90%, 95%, 96%, 97%, 98%, 99%, 99.5% or 99.9% identity to the above amino acid sequence.

In a preferred embodiment, the pyrimidine nucleoside phosphorylase mutant includes a protein that has greater than 75%, 80%, 85%, 90%, 95%, 96%, 97%, 98%, 99%, 99.5% or 99.9% identity to the amino acid sequence as defined in (a) and has a pyrimidine nucleoside phosphorylase activity.

All the above amino acid mutations have been experimentally investigated in the examples of this application. Compared with the parent having the amino acid sequence as shown in SEQ ID NO: 1, all of them have the activity of catalyzing the cleavage of a substrate nucleoside containing a 2'-fluoropentose structure into a phosphorylated 2'-fluoropentose and a free base. All the above mutation sites are mutations around the amino acid active site. Such mutations can improve the binding ability and/or catalytic ability of the mutant with the substrate. For mutations far from the active site, they have little impact on the catalytic ability of the enzyme. Therefore, a protein that has 70%, 75%, 80%, 85%, 90%, 95%, 96%, 97%, 98%, 99%, 99.5%, 99.9% or more identity to the above amino acid sequence and the same catalytic activity can be obtained.

"Identity" in this specification refers to the "identity" between amino acid sequences, i.e., the total ratio of amino acid residues of the same type in the amino acid sequences. The identity of amino acid sequences can be determined using alignment programs such as BLAST (Basic Local Alignment Search Tool), FASTA, and the like.

A protein that has greater than 70%, 75%, 80%, 85%, 90%, 95%, 99% (such as greater than 85%, 86%, 87%, 88%, 89%, 90%, 91%, 92%, 93%, 94%, 95%, 96%, 97%, 98%, 98.5%, 99%, 99.5%, 99.6%, 99.7%, 99.8%, and even greater than 99.9%) identity and the same function is highly likely to have the same active site, active pocket, active mechanism, protein structure, etc. as the protein provided by the sequence in (a), and is a homologous protein obtained through amino acid mutation.

As used herein, the abbreviations of amino acid residues are as follows: alanine (Ala; A), asparagine (Asn; N), aspartic acid (Asp; D), arginine (Arg; R), cysteine (Cys; C), glutamic acid (Glu; E), glutamine (Gln; Q), glycine (Gly; G), histidine (His; H), isoleucine (Ile; I), leucine (Leu; L), lysine (Lys; K), methionine (Met; M), phenylalanine (Phe; F), proline (Pro; P), serine (Ser; S), threonine (Thr; T), tryptophan (Trp; W), tyrosine (Tyr; Y), and valine (Val; V).

According to the rules of substitution and replacement, in general, the effect of mutual replacement between amino acids with similar properties is also similar. For example, in the above homologous proteins, conservative amino acid replacements may occur. "Conservative amino acid replacements" include but are not limited to:
Hydrophobic amino acids (Ala, Cys, Gly, Pro, Met, Val, Ile, Leu) substituted by other hydrophobic amino acids;
Hydrophobic amino acids with bulky side chains (Phe, Tyr, Trp) substituted by other hydrophobic amino acids with bulky side chains;
Amino acids with positively charged side chains (Arg, His, Lys) substituted by other amino acids with positively charged side chains; and
Amino acids with polar but uncharged side chains (Ser, Thr, Asn, Gln) substituted by other amino acids with polar but uncharged side chains.

A person skilled in the art can also perform conservative replacements of amino acids according to amino acid replacement rules well-known in the art, such as the "blosum62 scoring matrix" in the prior art.

"AlphaFold2-Multimer" used in this application is a publicly available artificial intelligence model capable of predicting the conformation of protein complexes. Its prediction of protein three-dimensional structure can be very close to the level observed with equipment such as cryo-electron microscopy in real experiments. It can obtain a relatively realistic protein structure, thereby guiding the exploration of protein structure and protein activity.

In a second typical implementation of this application, a DNA molecule is provided, where the DNA molecule encodes the above pyrimidine nucleoside phosphorylase mutant. In a third typical implementation of this application, a recombinant plasmid is provided, where the above DNA molecule is ligated to the recombinant plasmid.

The above DNA can encode the above pyrimidine nucleoside phosphorylase mutant, and can be ligated to the recombinant plasmid to form circular DNA. Both the above DNA and the recombinant plasmid are capable of undergoing transcription and translation under the action of RNA polymerase, ribosomes, tRNA, etc., to obtain the above pyrimidine nucleoside phosphorylase mutant.

In a fourth typical implementation of this application, a host cell is provided, where the host cell includes the above DNA molecule or recombinant plasmid.

In a preferred embodiment, the host cell includes a prokaryotic cell; and preferably, the prokaryotic cell includes *Escherichia coli.*

With the above host cell, the replication of the recombinant plasmid can be carried out in the host cell, and the DNA molecule carried on the recombinant plasmid can also be transcribed and translated to obtain a large amount of pyrimidine nucleoside phosphorylase mutants. With the prior art, such as cell disruption for protein purification of host cells, crude enzyme catalysis after disruption, or other methods, the pyrimidine nucleoside phosphorylase mutant can be obtained for subsequent catalysis of the substrate nucleoside. The host cell is a non-plant-derived host cell.

In a fifth typical implementation of this application, a method for preparing a 2'-fluoronucleoside is provided, where the method includes: a) catalyzing the cleavage of a substrate nucleoside into a phosphorylated 2'-fluoropentose and a free base with the above pyrimidine nucleoside phosphorylase mutant, where the substrate nucleoside has a 2'-fluoropentose structure; b) catalyzing the binding of the phosphorylated 2'-fluoropentose to a substrate base with a purine nucleoside phosphorylase, so as to obtain the 2'-fluoronucleoside.

In the above preparation method, the purine nucleoside phosphorylase includes but is not limited to a protein derived from *Geobacillus stearothermophilus* as shown in SEQ ID NO: 3 or a mutant thereof (with 80% or more identity), and also includes purine nucleoside phosphorylases (PNP) derived from other sources.

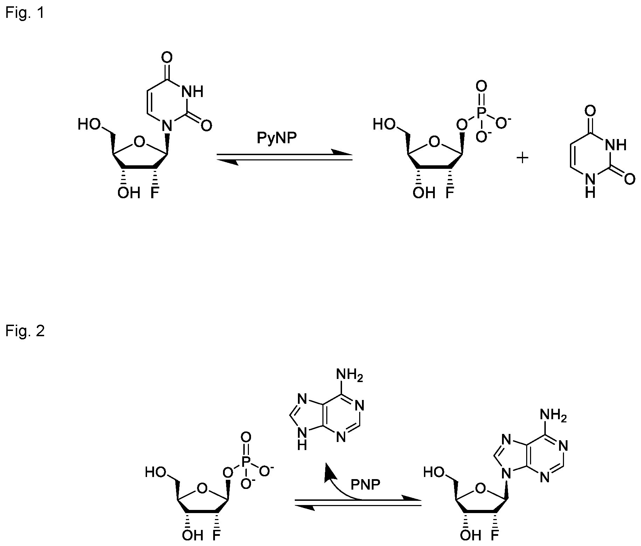

With the above preparation method, the above pyrimidine nucleoside phosphorylase mutant (PyNP) can be used to perform the following catalysis first, and the reaction schematic diagram is shown in Fig. 1. The substrate nucleoside is converted into a phosphorylated 2'-fluoropentose and a free base. The phosphate group in the phosphorylated 2'-fluoropentose is derived from the phosphate in the catalytic system. The structure of the substrate nucleoside includes but is not limited to the structure shown in Fig. 1.

Then, with the purine nucleoside phosphorylase (PNP) from the prior art, the substrate base is used to substitute the phosphate group on the phosphorylated 2'-fluoropentose, thereby obtaining the 2'-fluoronucleoside combined with the substrate base. The PNP used in this step can be flexibly selected from purine nucleoside phosphorylases in the prior art, all of which can catalyze the reaction. The reaction schematic diagram is shown in Fig. 2, and the structures of the substrate base and phosphorylated 2'-fluoropentose include but are not limited to those shown in Fig. 2.

Moreover, the two steps a) and b) can be carried out simultaneously at the same time, in the same container and under the same reaction conditions. Therefore, the above preparation method can realize the preparation of the 2'-fluoronucleoside by the one-pot method. The above preparation method has a higher conversion rate and greater efficiency, and can significantly reduce the amount of enzyme used, thereby lowering production costs, and is suitable for industrial scale-up production.

In a preferred embodiment, the substrate nucleoside includes a 2'-fluoro-2'-deoxyuridine, a 2'-fluoro-2'-deoxycytidine or a 2'-deoxy-2'-fluorothymidine.

In a preferred embodiment, the substrate base includes a modified or unmodified adenine, guanine, thymine or cytosine, or a modified uracil.

In a preferred embodiment, the 2'-fluoronucleoside includes a nucleoside containing a 2'-fluoro-2'-deoxypentose structure, and preferably, the 2'-fluoronucleoside includes a 2'-fluoro-2'-deoxyadenosine, a 2'-fluoro-2'-deoxy-2-aminoadenosine, a 2'-fluoro-2'-deoxyguanosine or a 2'-fluoro-2'-deoxycytidine; or the 2'-fluoronucleoside is selected from 2'-fluoro-2'-deoxynucleosides with substituents on the base structure.

The substituent on the above base structure or the modification of the above substrate base includes but is not limited to introduction of methyl (-CH₃), carbamoyl (-CH₂NH₂), glycosyl, a phosphonic acid group (-PO₄), thiol (-SH), nitro (-NO₂), hydroxyl (-OH) or deamination (-NH₂) on the base structure, thereby achieving modifications such as methylation, aminomethylation, glycosylation, phosphorylation, sulfuration, nitration, oxidation or deamination of the nucleoside.

Methylation: a modification of a base of a nucleoside by the addition of a methyl group. For example, in DNA, a cytosine base can undergo methylation to form 5-methylcytosine.

Aminomethylation: a modification of a base of a nucleoside by the addition of a carbamoyl group. This modification can occur on cytosine and adenine bases.

Glycosylation: a modification of a base of a nucleoside by the addition of a sugar moiety. For example, an adenosine in adenosine nucleotides can undergo glycosylation.

Phosphorylation: a modification of a base of a nucleoside by the addition of a phosphate group. Phosphorylation modification can occur on cytosine, adenine and guanine bases.

Sulfuration: a modification of a base of a nucleoside by the addition of a sulfur group. Sulfuration modification can occur on cytosine, adenine and guanine bases.

Nitration: a modification of a base of a nucleoside by the addition of a nitrate group. This modification can occur on cytosine and adenine bases.

Oxidation: a modification of a base of a nucleoside by the addition of an oxygen group. For example, in DNA, a guanine base undergoes oxidative modification to form 8-oxoguanine (8-oxoG).

Deamination: a modification of a base of a nucleoside by the loss of an amino group. This modification can occur on cytosine, adenine and guanine bases.

In the sixth typical implementation of the present application, a method for preparing a phosphorylated 2'-fluoropentose is provided, where the method includes: catalyzing the cleavage of a substrate nucleoside into a phosphorylated 2'-fluoropentose and a free base with the above pyrimidine nucleoside phosphorylase mutant, where the substrate nucleoside has a 2'-fluoropentose structure.

In a preferred embodiment, the substrate nucleoside includes a 2'-fluoro-2'-deoxyuridine, a 2'-fluoro-2'-deoxycytidine or a 2'-deoxy-2'-fluorothymidine.

The beneficial effects of this application will be further explained in detail below in conjunction with specific examples.

### Example 1 Construction of TtPyNP and GsPNP expression strains

The protein sequence of pyrimidine nucleoside phosphorylase derived from *Thermus thermophilus* (TtPyNP) (Accession No. BAD71594) was obtained from NCBI, as shown in SEQ ID NO: 1. After codon optimization, the DNA sequence encoding the enzyme was obtained, as shown in SEQ ID NO: 2, and cloned into the expression vector pET28a(+), resulting in the plasmid pET28a-TtPyNP. The pET28a-TtPyNP plasmid was transformed into *Escherichia coli* BL21(DE3) competent cells to obtain a monoclonal strain.

Using the same method, a pET28a-GsPNP (GsPNP, NCBI Accession No. BAA13510) monoclonal strain was obtained, and the GsPNP protein sequence is shown in SEQ ID NO: 3.
SEQ ID NO: 1: 
MNPVAFIREKREGKKHRREDLEAFLLGYLHDEVPDYQVSAWLMAAFLRGLDPEETLWLTETM
ARSGKVLDLSGLPHPVDQHSSGGVGDKVSLVVGPILAASGCTFAKMSGRTLAHTGGNIDKLESVP
GWRGEMTEAEFLERARRVGLVIAAQSPDLTPLDGKLYALRDVTATTESVPLIASSIMSKKLAVGAR
SIVLDVKVGRGAFMKTLEEARLLAKTMVAIGQGDGRRVRALLTSMEAPLGRAVGNAIEVREAIEAL
KGEGPGDLLEVALALAEEALRREGLDPALARKALEGGAALEKFRAFLEAQGGDPRAVEDFSLLPL
AEEHPLRAEREGVVREVDAYKVGLAVLALGGGRKRKGEPIDHGVGVYLLKKPGDRVERGEALAL
VYHRRRGLEEALGHLREAYALGEEAHPAPLVLEAI.
SEQ ID NO: 2: 
atgaacccggtggcgtttattcgcgaaaaacgcgaaggcaagaaacatcgccgcgaggatctggaagcgtttctgctgggctatct
gcacgatgaagtgccggattatcaggtgagcgcgtggctgatggcggcgtttttacgcggcttagatccggaagaaaccctgtggctgac
cgaaacgatggcccgcagcggtaaagtgctggatctgagcggtctgccgcatccggttgatcagcatagcagcggcggcgtgggcgat
aaagtgagcttagtggtgggcccgattctggcggcgagcggttgtacctttgcgaaaatgtcgggccgcaccctggcgcataccggtggta
acattgataaactggaaagcgtgccgggctggcgcggcgaaatgaccgaagcagaatttctggaacgcgcccgtcgcgttggtctggtt
attgcggcgcaaagcccggatttaacgccgttagatggcaaactgtatgcgctgcgcgatgtgaccgcgaccactgaatcagttccgctg
attgcgagcagcattatgagcaagaaactggcggtgggcgcccgtagcattgtgttagatgttaaagtgggccgcggcgcgtttatgaaa
accctggaagaagcgcgtctgctggcgaaaacgatggtggcgattggccagggcgatggccgtcgtgttcgtgcgttattaaccagcatg
gaagcgcctctgggtcgtgcggttggcaatgcgattgaagtgcgtgaagcgattgaagcgctgaaaggcgaaggcccgggcgatttatt
agaagtggcgctggcgttagcggaagaagcgctgcgtcgtgaaggcttagatccggcgctggcgcgtaaagcgttagaaggtggcgcg
gcgctggaaaaatttcgcgcgtttctggaagcgcagggcggcgatccgcgtgcagttgaagattttagcctgctgccgctggcggaagaa
catccgttacgtgcggaacgcgaaggcgtggttcgcgaagttgatgcgtataaagtgggcctggcggtgctggcgttaggcggtggtcgta
aacgtaaaggtgaaccgattgatcatggcgtgggcgtgtatctgctgaagaaaccgggcgatcgcgtggaacgcggcgaagcgttagc
gttagtgtatcatcgccgccgcggcttagaagaagcgctgggtcatttacgcgaagcgtatgcgctgggcgaagaagcgcatccggcgc
ctctggttttagaagcgatttaa.
SEQ ID NO: 3:

MSVHIGAKEHEIADKILLPGDPLRAKYIAETFLEGATCYNQVRGMLGFTGTYKGHRISVQGTG
MGVPSISIYITELMQSYNVQTLIRVGTCGAIQKDVKVRDVILAMTSSTDSQMNRMTFGGIDYAPTAN
FDLLKTAYEIGKEKGLQLKVGSVFTADMFYNENAQFEKLARYGVLAVEMETTALYTLAAKFGRKAL
SVLTVSDHILTGEETTAEERQTTFNEMIEVALETAIRQ.

### Example 2 Construction of TtPyNP mutants

Saturation mutagenesis or site-directed mutagenesis was performed by whole-plasmid PCR. The first round of mutagenesis used pET28a-TtPyNP obtained in Example 1 as the template, and subsequent mutagenesis used the optimal mutant screened in the previous round as the template. PCR reaction conditions were as follows: pre-denaturation at 94°C for 10 min; 25 amplification cycles (denaturation at 94°C for 30 s, and annealing and extension at 68°C for 6.5 min), and final extension at 68°C for 10 min. The obtained PCR product was digested with *Dpnl* enzyme and then transformed into *Escherichia coli* BL21(DE3) competent cells, followed by overnight culture at 37°C to obtain monoclonal colonies.

### Example 3 Expression of TtPyNP mutants and preparation of enzyme solution

Expression of mutants: the mutants were inoculated into a test tube containing 5 mL Luria-Bertani liquid medium (containing 50 µg/mL kanamycin), cultured at 37°C for 16 h, and then inoculated at a 1% inoculum volume into a 2 L shake flask containing 500 mL of Luria-Bertani liquid medium. When culturing at 37°C until OD₆₀₀ reached 0.6, IPTG was added at a final concentration of 0.1 mM to induce expression, followed by induction at 20°C for 18 h. The cultured bacterial solution was centrifuged at 7000 rpm for 10 min to collect the bacterial cells, which were stored at -20°C for later use.

Preparation of enzyme solution: 0.1 g of the bacterial sludge prepared above was weighed and resuspended in 1 mL of 100 mM potassium phosphate buffer (pH 7.5). After shaking and mixing, the bacterial cell suspension was disrupted with an ultrasonic disruptor (power: 30%, time: 5 min).

The expression of GsPNP and the preparation of its enzyme solution were performed as above.

### Example 4 HPLC detection method

HPLC detection method: chromatographic column: Atlantis T3 Column, 4.6 mm × 150 mm; mobile phase: 0.1% TFA/methanol; flow rate: 1 mL/min; 40°C; UV detector; detection wavelength: 254 nm; detection duration: 15 min. This HPLC condition can be used to detect the conversion rate of 2'-fluoro-2'-deoxyuridine.

### Example 5 Reaction verification of TtPyNP mutants

Single-point mutations and multi-point mutations were constructed using TtPyNP as the template, and reaction verification was performed. Reaction conditions: 20 mM 2'-fluoro-2'-deoxyuridine, 20 mM PBS (pH 7.0), 49 µL enzyme solution, total volume 1 mL, 60°C, reaction for 1 h. Samples were taken for HPLC detection, and the conversion rate of 2'-fluoro-2'-deoxyuridine is shown in Table 1. The results showed that the activity of mutants containing the K80 mutation site was significantly improved, and the conversion rate of the final mutant K80Q+G112T+T119N+A157T+V173T+M207L+A227D+L281R could reach greater than 30%.

**Table 1**

| Name | Conversion rate % |
|---|---|
| TtPyNP | + |
| V4G, V4P, 17G, R8F, R8N, R17E, E19P, G27S, R30K, P34G, P34K, P34L, L47H, G49S, G49T, G49V, L50A, L50D, L50G, L50K, L50N, L50R, L50S, L58Q, L58T, D61T, S91A, S91C, S91V, F105I, F105T, E135A, E135K, A151S, I179V, F206M, M207R, L215I, G224D, Q225N, V231D, A271S, R280V, G285D, R303A, R303K, D329F, V337T, G355S | + |
| K80Q, K80Y, G112A, G112C, G112L, G112S, G112T, T119N, A157D, A157N, A157T, V173S, V173T, I183V, A189T, A189V, A190I, A190T, A190V, M207L, A227D, L274I, L274K, L281R, L284G, L284S, K360I, K360L, K80Q+G112A, K80Q+G112C, K80Q+G112S, K80Q+G112T, L50A+K80Q+G112T, L50D+K80Q+G112T, L50G+K80Q+G112T, L50K+K80Q+G112T, L50N+K80Q+G112T, L50S+K80Q+G112T | ++ |
| K80Q+G112T+A157T, D61T+K80Q+G112T+A157T, E19P+K80Q+G112T+A157T, G27S+K80Q+G112T+A157T+L281R, G49S+K80Q+G112T+A157T, G49T+K80Q+G112T+A157T, G49V+K80Q+G112T+A157T, I7G+K80Q+G112T+A157T, K80Q+G112T+A157T+A190T+L281R, K80Q+G112T+A157T+A190V+L281R, K80Q+G112T+A157T+A271S+L281R, K80Q+G112T+A157T+L215I+L281R, K80Q+G112T+A157T+L274I+L281R, K80Q+G112T+A157T+L274K, K80Q+G112T+A157T+L281R, K80Q+G112T+A157T+L281R+G355S, K80Q+G112T+A157T+L281R+K360l, K80Q+G112T+A157T+L281R+V337T, K80Q+G112T+A157T+L284G, K80Q+G112T+A157T+L284S, K80Q+G112T+A157T+Q225N+L281R, K80Q+G112T+A157T+R280V, K80Q+G112T+A157T+R303K, K80Q+G112T+A157T+V173S, K80Q+G112T+A157T+V173T+A189V+L281R, K80Q+G112T+A157T+V173T+L281R, K80Q+G112T+A189V, | +++ |
| K80Q+G112T+A190I, K80Q+G112T+A190T, K80Q+G112T+E135A+A157T+L281R, K80Q+G112T+E135K, K80Q+G112T+K360I, K80Q+G112T+V173T, L47H+K80Q+G112T+A157T, L50A+K80Q+G112T+A157T+V173T+L281R, L50K+K80Q+G112T+A157T+L281R, L50N+K80Q+G112T+A157T+L281R, L50R+K80Q+G112T+A157T, L58Q+K80Q+G112T+A157T, L58T+K80Q+G112T+A157T, P34G+K80Q+G112T+A157T, P34K+K80Q+G112T+A157T, P34L+K80Q+G112T+A157T+L281R, R17E+K80Q+G112T+A157T, V4G+K80Q+G112T+A157T+L281R, V4P+K80Q+G112T+A157T, V4P+L50R+G112C+11S3V+A190T+L2151+L2S1R+K360L | |
| E19P+P34K+F105I+A157N+V173S+F206M+V231D+L281R, K80Q+G112T+A157T+V173T+A227D+L281R, K80Q+G112T+A157T+V173T+G224D+L281R, K80Q+G112T+A157T+V173T+L281R+D329F, K80Q+G112T+A157T+V173T+L281R+G285D, K80Q+G112T+A157T+V173T+L281R+R303A, L47H+L58Q+S91V+T119N+A157T+M207R+G224D+A271S, R17E+R30K+G112L+E135K+A157D+I183V+L284G+K360I, R30K+K80Q+G112T+A157T+V173T+L281R, R8F+G27S+S91A+G112T+A151S+V173T+M207L+V337T, V4G+R8N+P34L+G49V+L50D+V173T+L2151+G355S | ++++ |
| K80Q+G112S+1179V+A227D+L274K+R280V+R303A+K360L, K80Q+G112T+A157T+V173T+A189T+A227D+L281R, K80Q+G112T+A157T+V173T+A189V+A227D+L281R, K80Q+G112T+A157T+V173T+A190V+A227D+L281R, K80Q+G112T+A157T+V173T+F206M+A227D+L281R, K80Q+G112T+A157T+V173T+1179V+A227D+L281R, K80Q+G112T+A157T+V173T+I183V+A227D+L281R, K80Q+G112T+A157T+V173T+M207R+A227D+L281R, K80Q+G112T+T119N+A157T+V173T+A227D+L281R, K80Y+F105T+G112A+A157T+V173T+A189T+Q225N+L274I, L50A+K80Q+V173T+I183V+A189T+Q225N+L274I+G285D, L50G+K80Y+T119N+V173T+L215I+L284S+R303K+D329F | +++++ |
| K80Q+G112T+T119N+A157T+V173T+I179V+A227D+E251V, K80Q+G112T+T119N+A157T+V173T+I179V+A227D+L281R, K80Q+G112T+T119N+A157T+V173T+I183V+A227D+L281R, K80Q+G112T+T119N+A157T+V173T+A189T+A227D+L281R, K80Q+G112T+T119N+A157T+V173T+A189V+A227D+L281R, K80Q+G112T+T119N+A157T+V173T+A190T+A227D+L281R, K80Q+G112T+T119N+A157T+V173T+A190V+A227D+L281R, K80Q+G112T+T119N+A157T+V173T+F206M+A227D+L281R, K80Q+G112T+T119N+A157T+V173T+M207L+A227D+L281R | ++++++ |

| | |
|---|---|
| Note: in the above table, the "+" represents a conversion rate of 1-10% (excluding the endpoint value of 10%), the "++" represents a conversion rate of 10-15% (excluding the endpoint value of 15%), the "+++" represents a conversion rate of 15-20% (excluding the endpoint value of 20%), the "++++" represents a conversion rate of 20-25% (excluding the endpoint value of 25%), the "+++++" represents a conversion rate of 25-30%, and the "++++++" represents a conversion rate of >30%. | |

### Example 6 Use of TtPyNP mutants in synthesis of 2'-fluoro-2'-deoxyadenosine

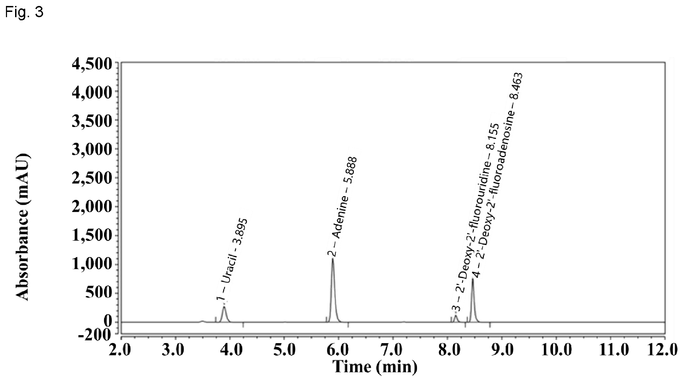

In a 20 mM phosphate buffer (pH 7.5), a 10 mL reaction system was prepared, which included two substrates: 100 mM 2'-fluoro-2'-deoxyuridine (2'-deoxy-2'-fluorouridine) and 250 mM adenine, two enzyme solutions: 1.07 mL of the mutant K80Q+G112T+T119N+A157T+V173T+M207L+A227D+L281R enzyme solution and 4.3 mL of GsPNP enzyme solution, 60°C, reaction for 137 h. After the reaction was completed, 1 mL of the reaction solution was taken and mixed with an equal volume of DMSO. After being diluted 25 times, the mixture was sent for HPLC detection. The HPLC chromatogram is shown in Fig. 3. The conversion rate of 2'-fluoro-2'-deoxyuridine was >80% (converted to uracil), the conversion rate of adenine was >30%, and the yield of 2'-fluoro-2'-deoxyadenosine (2'-deoxy-2'-fluoroadenosine) was >20 g/L.

From the above description, it can be seen that the above examples of the present application achieve the following technical effects: the substrate nucleoside containing a 2'-fluoropentose structure can be efficiently catalyzed to cleave into a phosphorylated 2'-fluoropentose and a free base with the above pyrimidine nucleoside phosphorylase mutant. Further, the 2'-fluoronucleoside can be prepared by the one-pot method with the above pyrimidine nucleoside phosphorylase mutant and the purine nucleoside phosphorylase in the prior art. Compared with the wild-type enzyme, the above pyrimidine nucleoside phosphorylase mutant has a better enzyme activity, and a higher conversion rate and greater efficiency in preparing 2'-fluoronucleoside products. It can significantly reduce the amount of enzyme used, thereby lowering production costs, and is suitable for industrial scale-up production.

The above descriptions are merely preferred examples of the present application and are not intended to limit the present application. For those skilled in the art, various modifications and changes may be made to the present application. Any modification, equivalent replacement, improvement, etc. made within the spirit and principles of the present application shall be included within the scope of protection of the present application.

## Claims

1. A pyrimidine nucleoside phosphorylase mutant, comprising:
(a) a protein with a mutation based on the pyrimidine nucleoside phosphorylase wild-type enzyme TtPyNP as shown in SEQ ID NO: 1, the mutation is selected from any one or more of the following mutations: K80, V4, 17, R8, R17, E19, G27, R30, P34, L47, G49, L50, L58, D61, S91, F105, G112, T119, E135, A151, A157, V173, I179, I183, A189, A190, F206, M207, L215, G224, Q225, A227, V231, E251, A271, L274, R280, L281, L284, G285, R303, D329, V337, G355 or K360; or
(b) a protein that has greater than 70% identity to the amino acid sequence as defined in (a) and has a pyrimidine nucleoside phosphorylase activity.

2. The pyrimidine nucleoside phosphorylase mutant according to claim 1, wherein in the (a), the amino acid substituted at each site is each independently selected from the followings:
K80Q, K80Y, V4P, V4G, I7G, R8N, R8F, R17E, E19P, G27S, R30K, P34K, P34L, P34G, L47H, G49T, G49V, G49S, L50A, L50D, L50G, L50K, L50N, L50R, L50S, L58T, L58Q, D61T, S91A, S91V, S91C, F1051, F105T, G112S, G112T, G112A, G112L, G112C, T119N, E135K, E135A, A151S, A157D, A157N, A157T, V173S, V173T, I179V, I183V, A189V, A189T, A190T, A190V, A190I, F206M, M207L, M207R, L2151, G224D, Q225N, A227D, V231D, E251V, A271S, L274K, L274I, R280V, L281R, L284S, L284G, G285D, R303K, R303A, D329F, V337T, G355S, K360L or K360I;
wherein, a letter before a number represents an original amino acid, and a letter after the number represents a mutated amino acid.

3. The pyrimidine nucleoside phosphorylase mutant according to claim 1, wherein the mutation comprises any one of the following amino acid mutations:
K80Q+V4P, K80Q+V4G, K80Q+I7G, K80Q+R8N, K80Q+R8F, K80Q+R17E, K80Q+E19P, K80Q+G27S, K80Q+R30K, K80Q+P34K, K80Q+P34L, K80Q+P34G, K80Q+L47H, K80Q+G49T, K80Q+G49V, K80Q+G49S, K80Q+L50A, K80Q+L50D, K80Q+L50G, K80Q+L50K, K80Q+L50N, K80Q+L50R, K80Q+L50S, K80Q+L58T, K80Q+L58Q, K80Q+D61T, K80Q+S91A, K80Q+S91V, K80Q+S91C, K80Q+F105I, K80Q+F105T, K80Q+G112S, K80Q+G112T, K80Q+G112A, K80Q+G112L, K80Q+G112C, K80Q+T119N, K80Q+E135K, K80Q+E135A, K80Q+A151S, K80Q+A157D, K80Q+A157N, K80Q+A157T, K80Q+V173S, K80Q+V173T, K80Q+I179V, K80Q+1183V, K80Q+A189V, K80Q+A189T, K80Q+A190T, K80Q+A190V, K80Q+A190I, K80Q+F206M, K80Q+M207L, K80Q+M207R, K80Q+L215I, K80Q+G224D, K80Q+Q225N, K80Q+A227D, K80Q+V231D, K80Q+A271S, K80Q+L274K, K80Q+L274I, K80Q+R280V, K80Q+L281R, K80Q+L284S, K80Q+L284G, K80Q+G285D, K80Q+R303K, K80Q+R303A, K80Q+D329F, K80Q+V337T, K80Q+G355S, K80Q+K360L, K80Q+K360I, K80Y+A190V, K80Y+A190I, K80Q+V4G+I183V, K80Q+V4P+L58Q, K80Q+I7G+R30K, K80Q+R8F+L281R, K80Q+R8N+G112A, K80Q+R17E+V337T, K80Q+E19P+L50G, K80Q+G27S+I179V, K80Q+P34G+A189T, K80Q+P34K+G49V, K80Q+P34L+E135K, K80Q+L47H+A271S, K80Q+G49S+A157N, K80Q+G49T+L284S, K80Q+L50A+V173T, K80Q+L50A+G112T, K80Q+L50D+G112T, K80Q+L50D+A157D, K80Q+L50G+G112T, K80Q+L50K+G112T, K80Q+L50K+G285D, K80Q+L50N+G112T, K80Q+L50N+V231D, K80Q+L50R+K360I, K80Q+L50S+G112T, K80Q+L50S+A189V, K80Q+L58T+F105I, K80Q+D61T+L284G, K80Q+S91A+F105T, K80Q+S91C+G112L, K80Q+S91V+A190V, K80Q+G112S+E135A, K80Q+G112C+R303A, K80Q+G112T+E135K, K80Q+G112T+A157T, K80Q+G112T+V173T, K80Q+G112T+A189V, K80Q+G112T+A190I, K80Q+G112T+A190T, K80Q+G112T+K360I, K80Q+G112T+K360L, K80Q+T119N+A157T, K80Q+A151S+G224D, K80Q+V173S+L215I, K80Q+A190I+F206M, K80Q+A190T+A227D, K80Q+M207R+Q225N, K80Q+M207L+L274K, K80Q+L274I+G355S, K80Q+R280V+R303K, K80Q+V4G+D61T+M207R, K80Q+V4P+S91V+R280V, K80Q+V4P+G112T+A157T, K80Q+17G+L50N+F105T, K80Q+I7G+G112T+A157T, K80Q+R8F+A157D+L215I, K80Q+R17E+L50S+L274K, K80Q+R17E+G112T+A157T, K80Q+E19P+G112T+A157T, K80Q+E19P+A157N+L284S, K80Q+G27S+R30K+L58Q, K80Q+P34G+G112S+A271S, K80Q+P34G+G112T+A157T, K80Q+P34L+I179V+V337T, K80Q+P34K+G112T+A157T, K80Q+P34K+I1S3V+L2S1R, K80Q+L47H+S91A+V173T, K80Q+L47H+G112T+A157T, K80Q+G49S+L50R+A151S, K80Q+G49V+E135K+D329F, K80Q+G49V+G112T+A157T, K80Q+G49T+F105I+K360I, K80Q+G49T+G112T+A157T, K80Q+G49S+G112T+A157T, K80Q+L50K+A157T+G285D, K80Q+L50G+F206M+G355S, K80Q+L50R+G112T+A157T, K80Q+L58T+G112T+A157T, K80Q+L58T+A1901+A189T, K80Q+L58Q+G112T+A157T, K80Q+D61T+G112T+A157T, K80Q+S91C+G112C+V173S, K80Q+G112A+T119N+M207L, K80Q+G112L+A190V+V231D, K80Q+G112L+V173S+A190V, K80Q+G112T+A157T+L274K, K80Q+G112T+A157T+L281R, K80Q+G112T+A157T+R303K, K80Q+G112T+A190T+G224D, K80Q+E135A+L284G+R303A, K80Q+A189V+A227D+R303K, K80Q+L2741+Q225N+K360L, K80Q+V4G+P34L+L50N+L284S, K80Q+V4G+G112T+A157T+L281R, K80Q+V4P+17G+R30K+F206M, K80Q+R8N+G112C+T119N+L284G, K80Q+R8F+F105I+G112S+A157T, K80Q+R17E+L50S+I1S3V+L274I, K80Q+E19P+L50G+V173T+K360I, K80Q+G27S+G112T+A157T+L281R, K80Q+G27S+A190T+L274K+G355S, K80Q+P34L+G112T+A157T+L281R, K80Q+P34K+G112T+I179V+R303A, K80Q+L47H+A190V+M207R+A227D, K80Q+G49S+L50R+V231D+D329F, K80Q+G49T+S91A+G112T+V337T, K80Q+G49V+F105T+A271S+K360L, K80Q+L50K+L58T+A151S+M207L, K80Q+L50A+G112A+A157T+Q225N, K80Q+L50K+G112T+A157T+L281R, K80Q+L50N+G112T+A157T+L281R, K80Q+L50D+A157N+A189V+A227D, K80Q+L58Q+D61T+G112L+G224D, K80Q+S91C+A157T+L2151+L281R, K80Q+S91V+V173S+A190I+R303K, K80Q+G112L+E135A+A189V+L281R, K80Q+G112T+A157T+L274I+L281R, K80Q+G112T+A157T+V173T+L281R, K80Q+T119N+E135A+L274K+G285D, K80Q+E135A+A157T+A227D+K360I, K80Q+V4G+L50S+L58T+F206M+A271S, K80Q+V4P+L50G+A151S+D61T+L284G, K80Q+17G+L50D+S91V+A189V+D329F, K80Q+R8N+L50R+G112C+A157T+A190T, K80Q+R8F+G49T+S91C+Q225N+G355S, K80Q+R17E+E135A+A157N+I183V+K360L, K80Q+E19P+L50S+A157T+A190V+G112T, K80Q+G27S+L50A+G112S+A227D+L281R, K80Q+R30K+L58Q+T119N+A157D+L281R, K80Q+R30K+G112T+A157T+V173T+L281R, K80Q+P34G+V173S+A190I+M207R+R303K, K80Q+P34L+L47H+F105T+R280V+R303A, K80Q+P34K+G112L+E135K+A189T+L215I, K80Q+P34K+G112T+I179V+L281R+R303A, K80Q+G49V+A157T+M207L+L2741+G285D, K80Q+G49S+F1051+I179V+G224D+L284S, K80Q+S91A+V173T+L274K+V337T+K360L, K80Q+L50A+G112T+A157T+V173T+L281R, K80Q+G112L+V173T+A190V+A227D+K360I, K80Q+G112T+A157T+V173T+A189V+L281R, K80Q+G112T+A157T+V173T+L281R+G285D, K80Q+V4G+S91V+G112L+E135K+A151S+G224D, K80Q+V4P+L50D+S91A+A189T+A227D+L274K, K80Q+V4P+G112A+V173T+V231D+L281R+V337T, K80Q+I7G+G27S+L47H+S91C+A189V+L2151, K80Q+R8F+T119N+A157N+L281R+G355S+K360L, K80Q+R8N+E135A+A157D+V173T+F206M+K360I, K80Q+R17E+P34L+G49T+G112S+I179V+A190T, K80Q+E19P+G49S+A157T+A189V+A227D+G285D, K80Q+R30K+L50A+V173T+A190V+Q225N+L281R, K80Q+P34G+L50K + T119N+M207R+A227D+L284S, K80Q+P34K+G112T+E135K+A189T+L215I+L274K, K80Q+P34K+F105I+T119N+A189V+R280V+D329F, K80Q+L50R+T119N+A190I+M207L+R303A+K360I, K80Q+L50S+D61T+V157T+I183V+L274I+L281 R, K80Q+G112T+T119N+A157T+V173T+A227D+L281R, K80Q+G112T+E135K+A157T+V173T+L215I+L281R, K80Q+G112T+A157T+V173T+A189V+A227D+L281R, K80Q+V4G+R8N+L58T+G112C+E135K+A190T+R303K, K80Q+V4P+L58Q+G112L+A190I+M207L+V231D+K3601, K80Q+R8F+P34K+G49T+T119N+V173T+A190V+L274K, K80Q+P34K+L50R+S91C+G112T+I179V+G224D+G355S, K80Q+L47H+S91V+E135A+V173S+F206M+R280V+V337T, K80Q+R17E+A151S+A157N+V173T+V231D+R280V+K360L, K80Q+I7G+G49V+S91C+F105T+G112A+L281R+R303A, K80Q+E19P+G27S+P34L+L50G+F105I+A271S+L281 R, K80Q+G49S+D61T+A189V+F206M+A227D+L284S+D329F, K80Q+P34G+L50A+A157T+I183V+M207R+D329F+K360L, K80Q+L50D+S91A+G112S+L215I+Q225N+L274I+G285D, K80Q+P34K+G112T+E135K+A189T+L215I+L274K+K360I, K80Q+P34K+G112T+I179V+A189V+A227D+L281R+R303A, K80Q+L50D+T119N+A157T+V173T+A190V+A227D+L281R, K80Q+G112T+T119N+A157T+V173T+A190V+A227D+L281R, K80Q+G112T+A157T+V173T+A189V+M207R+A227D+L360L, K80Q+17G+L50A+G112A+E135A+A157D+M207L+A227D+D329F, K80Q+R8N+G27S+L50R+D61T+G112C+A189V+G224D+L281R, K80Q+R17E+L47H+D61T+A189V+L215I+A227D+L274K+R303K, K80Q+E19P+P34K+G49T+I183V+A190I+M207R+G285D+K360I, K80Q+P34G+G49V+L58Q+S91C+G112C+A151S+L284G+V337T, K80Q+P34K+G112T+A157T+V173T+A190T+F206M+A227D+R280V, K80Q+L50G+S91A+F105T+E135K+V173S+L215I+R280V+R303K, K80Q+S91V+A157N+Q225N+A271S+L284S+R303A+G355S+K360I, K80Q+L50D+T119N+A157T+V173T+A190V+A227D+L281R+K360I, K80Q+G112T+T119N+A157T+V173T+A190V+A227D+L281R+K360I, K80Q+V4G+P34K+L50A+F105I+G112T+A157N+A190V+L281R+R303A, K80Q+V4P+R8N+L47H+S91A+A157D+V173S+I183V+A190T+G285D, K80Q+I7G+E19P+G27S+S91A+G224D+L274K+R280V+R303K+D329F, K80Q+R8F+P34G+L50K+G112C+A157T+A189T+A227D+L281R+K360I, K80Q+R17E+P34L+G49T+G112L+M207L+E135K+Q225N+L284S+K360I, K80Q+P34K+L50D+G112A+A151S+A157D+I179V+A1901+F206M+V337T, K80Q+P34K+L58Q+D61T+F105T+E135A+L2151+M207R+A271S+G355S, K80Q+P34K+L58Q+G112T+T119N+A157T+V173T+A190V+A227D+L281R, K80Q+P34K+L58Q+G112T+I179V+A189V+A227D+L281R+R303A+K360L, K80Q+L50R+S91V+G112T+T119N+V173T+V231D+L274I+L284G+K360L, K80Q+L50D+T119N+A157T+V173T+A189V+A190V+A227D+L281R+K360I, or K80Q+G112T+T119N+A157T+V173T+A190V+A227D+L281R+R303A+K360I.

4. A DNA molecule, wherein the DNA molecule encodes the pyrimidine nucleoside phosphorylase mutant according to any one of claims 1 to 3.

5. A recombinant plasmid, wherein the DNA molecule according to claim 4 is comprised in the recombined plasmid.

6. A host cell, wherein the host cell comprises the DNA molecule according to claim 4 or the recombinant plasmid according to claim 5.

7. A method for preparing a 2'-fluoronucleoside, wherein the method comprises:
a) catalyzing the cleavage of a substrate nucleoside into a phosphorylated 2'-fluoropentose and a free base with the pyrimidine nucleoside phosphorylase mutant according to any one of claims 1 to 3, wherein the substrate nucleoside has a 2'-fluoropentose structure;
b) catalyzing the binding of the phosphorylated 2'-fluoropentose to a substrate base with a purine nucleoside phosphorylase, so as to obtain the 2'-fluoronucleoside.

8. The method according to claim 7, wherein the substrate nucleoside comprises a 2'-fluoro-2'-deoxyuridine, a 2'-fluoro-2'-deoxycytidine or a 2'-deoxy-2'-fluorothymidine.

9. The method according to claim 7, wherein the substrate base comprises a modified or unmodified adenine, guanine, thymine or cytosine, or a modified uracil.

10. The method according to any one of claims 7 to 9, wherein the 2'-fluoronucleoside comprises a nucleoside comprising a 2'-fluoro-2'-deoxypentose structure.

11. The method according to claim 10, wherein the 2'-fluoronucleoside comprises a 2'-fluoro-2'-deoxyadenosine, a 2'-fluoro-2'-deoxy-2-aminoadenosine, a 2'-fluoro-2'-deoxyguanosine or a 2'-fluoro-2'-deoxycytidine;
or the 2'-fluoronucleoside is selected from a 2'-fluoro-2'-deoxynucleoside with a substituent on the base structure.

12. A method for preparing a phosphorylated 2'-fluoropentose, wherein the method comprises:
catalyzing the cleavage of a substrate nucleoside into a phosphorylated 2'-fluoropentose and a free base with the pyrimidine nucleoside phosphorylase mutant according to any one of claims 1 to 3, wherein the substrate nucleoside has a 2'-fluoropentose structure.

13. The method according to claim 12, wherein the substrate nucleoside comprises a 2'-fluoro-2'-deoxyuridine, a 2'-fluoro-2'-deoxycytidine or a 2'-deoxy-2'-fluorothymidine.
